# EUROPEAN PATENT APPLICATION

(11) **EP 1 422 651 A2**
(43) Date of publication of application: **26.05.2004**
(21) Application number: 03396105.3
(22) Date of filing: 20.11.2003
(51) Int. Cl.: G06F 19/00

(54) **Method and system for collecting patient feedback**

(30) Priority: 21.11.2002 FI 20022078
(71) Applicant: Arieste Oy, 90100 Oulu (FI)
(72) Inventor: Linden, Teppo, 90520 Oulu (FI); Junttila, Markus, 90150 Oulu (FI)
(74) Representative: Pykälä, Timo Tapani

(57) **Abstract**

The invention relates to a method of collecting patient feedback and to a system of collecting patient feedback. The method comprises inputting (202) a patient's telecommunication connection identifier into a hospital data system and specifying (204) an enquiry for the patient in the hospital data system. This is followed by receiving (206) the patient's reply to the enquiry and the telecommunication connection identifier in a feedback data system, and transferring (208) the reply and the telecommunication connection identifier from the feedback data system to the hospital data system. The final step comprises combining (210) the patient's reply with the enquiry in the hospital data system, based on the telecommunication connection identifier.

## Description

### FIELD

The invention relates to a method of collecting patient feedback and to a system of collecting patient feedback.

### BACKGROUND

In the early 1990's, the quality of services started to attract attention in Finnish health care. Organizations initiated quality projects and produced quality criteria for controlling their operation. Feedback from patients is one quality meter. Inquiries and interviews are carried out for this purpose. However, to acquire and process feedback is laborious even if tabulation and analysis programs were in use. In organizations, quality work is often carried out alongside with the basic work, and thus is may seem hard and arduous.

The quality management recommendation from the year 1995 stated that the focus of quality management should be customer orientation. The recommendation emphasizes the expertise of the client as an evaluator of the services rendered and the results thereof. Client analysis and a working feedback data system, for example, were among the means brought forth. The recommendation revised in the year 1999 emphasizes client participation.

In their national recommendation, the Ministry of Social Affairs and Health, the National Research and Development Centre for Welfare and Health (Stakes) and the Association of Finnish Local and Regional Authorities bring forth the following points of view as regards client participation in quality management. Health care organizations should provide inhabitants of municipalities, civil groups, clients, patients and relatives with a chance to participate actively in the evaluation of service quality at different levels. Operational systems should include structures and channels for clients to produce developmental propositions, to bring forth their needs and the problems they have encountered.

Patient feedback is an important sector in the study of the quality of treatment. The feedback indicates how a patient experienced the treatment received. It provides health care organizations with the chance to develop their operations to better serve the needs of a patient. For its part, it yields information about the situation and development needs in the different sectors. Customer orientation is a value controlling the operation of health care professionals, and the chance to give feedback about one's own needs, expectations and experience is emphasized as the most important quality aspect in health care.

According to the national recommendation, quality management is subject to systematic and reliable collection of information describing the ongoing operation. It emphasizes the production of high-quality real-time information. Enquiries are usually carried out by using questionnaires. At the present moment, one of the most all-round feedback data systems can be found in the social and health services of the Kuusamo municipality. There, feedback can be given in written form, via email and using a WWW server form. The personnel also document verbal feedback obtained spontaneously from clients. Original feedback is filed in paper form and the units evaluate it once weekly. However, the system is mainly based on randomly received feedback.

The regulations regarding data requiring special protection, to which patient data also belong, are as follows: Data processing and storage spaces for the data are to be sufficiently protected and monitored. Access to data systems is controlled, and unauthorized access thereto is inhibited by available means. The data are used, modified and otherwise processed only by authorized persons. User rights are appropriately restricted and the usage is sufficiently controlled. Information about the material is handed over only by people who are responsible for attending thereto. Information to be transferred is encrypted in data networks as required.

### BRIEF DESCRIPTION

The object of the invention is to provide an improved method of collecting patient feedback and an improved system for collecting patient feedback.

An aspect of the invention relates to a method of collecting patient feedback. The method comprises: inputting a patient's telecommunication connection identifier into a hospital data system; specifying an enquiry for the patient in the hospital data system; receiving the patient's reply to the enquiry and the telecommunication connection identifier in a feedback data system; transferring the reply and the telecommunication connection identifier from the feedback data system to the hospital data system; and combining the patient's reply with the enquiry in the hospital data system, based on the telecommunication connection identifier.

An aspect of the invention relates to a system for collecting patient feedback. The system comprises: a hospital data system configured to store a patient's telecommunication connection identifier and an enquiry to the patient; and a feedback data system configured to receive the patient's reply to the enquiry and the telecommunication connection identifier, and to transfer the reply and the telecommunication connection identifier to the hospital data system; and the hospital data system is further configured to combine the patient's reply with the enquiry, based on the telecommunication connection identifier.

An aspect of the invention relates to a method of collecting patient feedback. The method comprises: inputting a patient's telecommunication connection identifier in a hospital data system; specifying an enquiry for the patient in the hospital data system; generating an enquiry identifier for the patient's enquiry in the hospital data system; transferring the patient's telecommunication connection identifier and the enquiry identifier from the hospital data system to a feedback data system; transmitting an enquiry reminder including at least part of the enquiry identifier from the feedback data system to the patient by using a telecommunication connection corresponding to the telecommunication connection identifier; receiving the patient's reply and at least part of the enquiry identifier in the feedback data system; transferring the reply and the enquiry identifier from the feedback data system to the hospital data system; and combining the patient's reply with the enquiry in the hospital data system, based on the enquiry identifier.

An aspect of the invention relates to a system for collecting patient feedback. The system comprises: a hospital data system configured to store a patient's telecommunication connection identifier and an enquiry to the patient; to generate and store an enquiry identifier for the patient's enquiry, and to transfer the patient's telecommunication connection identifier and the enquiry identifier to a feedback data system; a feedback data system configured to transmit an enquiry reminder including at least part of the enquiry identifier to the patient by using a telecommunication connection corresponding to the telecommunication connection identifier, and to receive the patient's reply to the enquiry and at least part of the enquiry identifier, and to transfer the reply and the enquiry identifier to the hospital data system; and the hospital data system is further configured to combine the patient's reply with the enquiry, based on the enquiry identifier.

The method of the invention brings forth a plurality of advantages. It reduces problems related to the collection and analysis of patient feedback. It utilizes electronic media for obtaining the feedback, and allows processed data to be obtained for viewing by means of a separate user interface.

### LIST OF THE FIGURES

In the following, preferred embodiments of the invention will be described in detail with reference to the accompanying drawings, in which
Figure 1A and 1B are simplified block diagrams showing a system for collecting patient feedback, Figure 1A illustrating its general structure and Figure 1 B illustrating an embodiment thereof;
Figure 2 is a flow diagram illustrating a method of collecting patient feedback; and
Figure 3 illustrates the database structure of the system.

### DESCRIPTION OF EMBODIMENTS

First, basic concepts to be employed will be described.

**ASCII** (American Standard Code for Information Interchange) is a character code specifying the code numbers used to store characters in the memory and files of a computer.

**FTP** (File Transfer Protocol) is an Internet file transfer program and a related transfer protocol.

HTML (Hypertext Markup Language) is a markup language employed to describe the contents of WWW pages.

**Internet** is the world's largest computer network including tens of millions of computers and about 450 million users in the year 2001. The network is based on the TCP/IP transfer protocol.

**Intranet** employs the Internet technology in the internal communication and data systems of a company or organization.

An **IP address** is a 32-bit address of a computer in a TCP/IP network, expressed in written text with four numbers (0...255) separated by dots. For example, 193.35.120.5 identifies one given machine.

**Java™** is a programming language and a system-independent processing environment.

In a clinic, at least one procedure is performed on patients.

An enquiry is carried out to receive patient feedback. An enquiry may include treatment instructions or reminders.

An enquiry reminder is a message that a patient receives after a given time after the procedure.

A questionnaire is a paper form that the patient receives and takes along from a hospital after a procedure. For feedback, it contains an enquiry, reply alternatives and reply instructions.

A form is a summary made based on the replies given by patients.

A physician performs a procedure on a patient and browses the feedback.

Feedback is a reply given by a patient to an enquiry.

A firewall is a control program that monitors and restricts telecommunication between the internal local area network of a company or organization and the Internet.

A **server** is a computer in a local area network or data network, and is specialized in serving network workstations by providing them with services relating to file management, printing, telecommunication or the like. A server may also refer to a computer program receiving data from client programs and replying in an agreed manner to the data requests transmitted by them. Several server programs may run simultaneously in the same machine, e.g. an email server, a WWW server and a database server.

A protocol is a set of jointly agreed regulations and standards by the use of which two devices are able to transfer data between them.

A patient inputs an evaluation of the success of a procedure.

RMI (Remote Method Invocation) is a subprogram to be called over a network.

Feedback can be performed by physicians, supervisors, and, anonymously, researchers.

SSH (Secure Shell) is a protocol ensuring an encrypted data line over a network that is unreliable as regards information security.

SMS (Short Message Service) is a short message transmission service.

SMSC (Short Message Service Centre) is a centralized text message processing service provided by service providers. Visible to telephones as a number and to data systems as an URL.

**TCP/IP** (Transmission Control Protocol / Internet Protocol) is a protocol suite used in the Internet and enabling computers to transfer data to each other. The TCP layer (transport layer) divides the data to be transferred into packets, after which the IP layer attends to packet switching over a network to the correct address.

A procedure is a short stay surgical procedure.

A researcher is a person outside the hospital, who is able to process the feedback statistically.

URL (Uniform Resource Locator) is an identifying mechanism individualizing the protocol, the name of the server, the directory structure, and the individual file employed.

A supervisor is an authority allowed to study the successes of the physicians and procedures.

A reply is a patient's evaluation about the success of a procedure.

WWW (World Wide Web) is a service operating in the Internet, enabling easy and inexpensive distribution of text and images to the users of the network. The material is divided into pages placed at a WWW server as usual files and directories.

XML (Extensible Markup Language) is a language development for the specification of structured documents. It is used for a more exact description of the data on a WWW page.

An administrator is a person entitled to add and delete different users to and from a system, change their rights and create new enquiries.

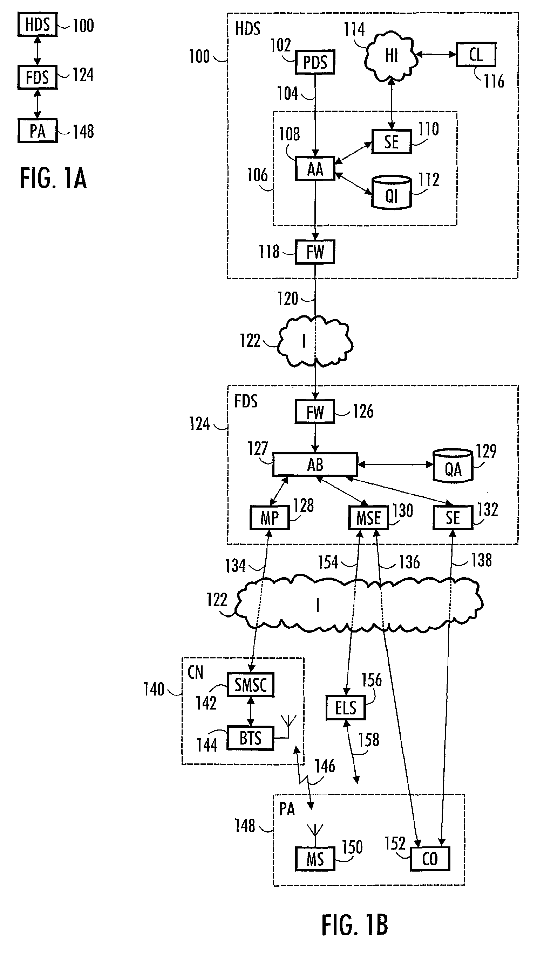

The basic concepts being now explained, the system for collecting patient feedback will be described with reference to Figure 1A. The two main parts of the system include a hospital data system 100 and a feedback data system 124. The hospital data system 100 is configured to store a patient's telecommunication connection identifier and an enquiry to a patient. A patient replies to the enquiry by using a selected data transfer device 148. The feedback data system 124 is configured to receive the patient's reply to the enquiry and the telecommunication connection identifier, and to transfer the reply and the telecommunication connection identifier to the hospital data system 100. The hospital data system 100 is configured to combine the patient's reply with the enquiry, based on the telecommunication connection identifier.

For information security reasons, data transfer between the hospital data system 100 and the feedback data system 124 can be implemented by configuring the hospital data system 100 to initiate data transfer with the feedback data system 124 by using a secure and/or encrypted data connection. The secure connection can be for instance a fixed connection between the hospital data system 100 and the feedback data system 124. Encryption can be implemented using encryption devices, particularly if the data connection passes via the Internet.

In an embodiment, the hospital data system 100 is also configured to transfer the patient's telecommunication identifier to the feedback data system 124. This being so, the feedback data system 124 is able to check if the reply it received is related to an enquiry. The feedback data system 124 can be configured to transmit an enquiry reminder to the patient using a telecommunication connection according to the telecommunication connection identifier. The feedback data system 124 can be configured to add, to the enquiry reminder, a link to the web page comprising the enquiry.

The contents of the enquiry reminder can be generated either in the feedback data system 124 or in the hospital data system 100. In the latter embodiment, the hospital data system 100 is configured to transfer the contents of the enquiry reminder to the feedback data system 124, and the feedback data system 124 is configured to receive the contents of the enquiry reminder from the hospital data system 100.

A patient's telecommunication connection identifier may be e.g. an email address, a radio system subscriber number, such as a mobile telephone number, or the identifier of another electronic medium, enabling unambiguous and global identification of the patient. Thus, the data transmission device 148 used by the patient can be for instance a computer connected to the Internet or mobile telephone.

If the data transmission device 148 is a computer, then the patient is able to answer the enquiry on a web page. This being so, the structure of the web page link is dynamic and individually generated for each patient. The feedback data system 124 is configured to receive the patient's reply from the web page. If the telecommunication connection identifier is an email address, then the feedback data system 124 is configured to receive the patient's reply in an email message. If the telecommunication connection identifier is a radio system subscriber number, then the feedback data system 124 is configured to receive the patient's reply in a short message.

Since, at least at the beginning, the system is new to the patient, reply instructions should be delivered to the patient. The hospital data system 100 can be configured to print out the enquiry, reply alternatives and reply instructions. These may then be given to the patient to take along when the patient visits the hospital. The feedback data system 124 can also be configured to deliver, to the patient, an address to the web page where the enquiry, reply alternatives and reply instructions are found. This delivery can take place to an address given by the patient and defined by the telecommunication connection identifier.

To ensure that a minimal amount of information is transferred by the use of telecommunication connections, for both cost reasons and particularly for information security reasons, the hospital data system 100 can be configured to generate the enquiry such that the enquiry is composed of multiple choices, and the reply is generated by symbols identifying the selected reply alternatives.

If in his reply a patient requests to be contacted, then the hospital data system 100 is automatically able to generate a message based on this to the person concerned in order for the matter to be processed, for instance by making an appointment or reserving a telephone time for the patient. In the system, automatic interactive feedback towards the patient can also be implemented, wherein for instance instructions for care can be delivered to the patient by using email. An email message can include instructions for care or a link to a WWW site containing the instructions for care. The link can also be transmitted in a short message.

The system is a tool for a hospital, clinic, nursing unit or an individual health care actor allowing the quality of the health care services of a particular sector, for example, to be monitored and developed. It enables cost-efficient large-scale patient follow-up. It is also applicable to conservative fields, for instance to the follow-up of new treatment.

In the present situation, the aim in special health care is to reduce the number of control visits. This has resulted in a situation wherein for instance after short stay surgical operations, the operating surgeon does not receive information about the long-term results of the treatment except in exceptional cases. The system gives extensive information about the success of a procedure in the short and long run to the performer of the procedure and to the nursing unit. This enables for instance a change in treatment practice when required. The system informs the treating unit about the patients that should be called in for check-up.

The different user groups of the system include: patient, processor of feedback (usually a physician), and administrator. The role of the patient is to answer the enquiry in accordance with given instructions by using the selected data transmission device 148. The processor of the feedback has access, within given limits, to the hospital data system 100 for studying the feedback, for instance as follows: monitor feedback, browse forms and search the replies for analysis results, for instance mean values. He logs on to the service using his user id and an alterable password. Ids and rights are given by the administrator. The processor of feedback usually logs on to the system from the hospital's internal network by entering his user id and the alterable password.

The tasks of an administrator include for instance the following: creation of enquiries (may vary according to patient group), monitoring event log, specification of users (addition, modification, erasure), specification of clinics and procedure classes (addition, modification, erasure). To add a user, the following data are required: user id, password, first name, last name, clinic and health insurance number. When a user is erased, the data on the user are maintained in the system, but he is no longer able to log on to the system. The administrator may modify the user data (password, last name, clinic). The administrator creates an enquiry by adding thereto the questions he deems appropriate, and, when needed, questions he creates. The administrator modifies an enquiry by deleting or adding questions from or to it. The administrator adds a procedure to the system, after which enquiries can be created for the procedure. The administrator adds, to the system, the clinic the patient feedback of whose procedures is to be monitored. The administrator generally monitors and controls the operation of the system.

The system is constructed for the use of the Oulu University Hospital (Oys) in the manner described in Figure 1 B, but the service can be adapted, with minor work, to correspond to the needs of other hospitals, too. At the initial stage at Oys, patients of the short stay surgery participate in collecting patient feedback. In the future, patients groups of other clinics will join the system.

The hospital data system 100 comprises the hospital's special patient management system 102. At Oys, the patient management system 102 is known by the name SAPO, wherein are located for instance appointments, personal data, address data, operation lists, treatment notices (HILMO), and final operation programs (LESU). The patient management system 102 is used via the hospital's Intranet 114 with terminals/micro workstations 116. A HILMO is filled in for every patient treated at the hospital. The HILMO includes the main events and information of the treatment, e.g. referral data, clinic, department, diagnosis, procedures, hospital discharge data and outpatient data. The HILMO is completed at the end of the treatment and serves as the basis of billing. The information contained by the HILMO is transferred to the Stakes register. A LESU is a patient's final operation schedule including personal data related to the procedure, the anaesthetic risk classification specified for the patient, for example.

The system is subject to minor changes in the existing patient management system 102. In the database of the patient management system 102, entry spaces are added to missing data (telecommunication connection identifier and the patient's consent to the enquiry). In addition, an analysis server (also known as Arieste server) 106 that has a telecommunication connection 104 to the patient management system 102 and is located in hospital premises, inside information security systems, is required, and also an entirely new feedback data system 124. A database search is implemented in the patient management system 102, enabling the transfer of the data of the patients participating in the enquiry to an AriesteA application 108 that is located in the analysis server 106 and constitutes a communication centre type of program entity located at the hospital. The system implemented at Oys is thus physically located in two places: in the hospital data system 100 located at the hospital, and in the feedback data system 124 located outside the hospital.

Data transmission 104 between the patient management system 102 and the analysis server 106 is implemented as follows. From the patient management system 102, the following data are picked into a transfer file: Specialty / clinic. Surgeon (last name_first name_second name or a five-character abbreviation generated by taking the first three characters of the last name and the first two characters of the first name, i.e. for instance a five-character abbreviation FINSE is generated for surgeon "Finne Seppo"). Social security number of patient. Sex of patient (F=female/M=male). Patient's telecommunication connection identifier. Diagnosis (according to the ICD 10 diagnosis list; there may be several, e.g. two, of which the first field is obligatory). Procedure (according to the ICD 10 diagnosis list; there may be several, e.g. three, of which the first field is obligatory). Anaesthesia classification (one character, value between 1 and 5). Date of procedure (in the form yyyymmdd, i.e. four digits for the year, two digits for the month and two digits for the day). Discharge date (in the form yyyymmdd, a single hyphen indicating that patient was discharged immediately).

The agreed conditions for picking patient data are that the patient participates in the enquiry, the patient has had surgery and has left the hospital (been discharged). The data are transmitted by utilizing an FTP transmission connection. The transfer takes place once daily, the transfer file is called coyyyymmdd.txt and it is an ASCII file. The semicolon ; operates as field separator. The line feed character separates the patients from each other, and <eof> (end of file) is the end sign of the transmission.

The file could be for instance co20020323.txt, its contents being: Kir1;FINSE;110673-XXX;M;pot@org.fi;D21.1;NRC99;1; 20020322;-<eof>.

The connection 120 between the AriesteA application 108 and the AriesteB application 127 is one-way. No connection to the hospital can be established from the AriesteB application 127. When transmitting patients' connection data and possibly an enquiry to the AriesteB application 127, the AriesteA application 108 simultaneously asks for patients' replies. The RMI connection 120 between the applications 108, 127 is protected by the SSH method. A tunnel protected by encryption is created on the connection 120 from the analysis server 106 to the feedback data system 124, making it extremely difficult for an incidental listener to find out the data carried in the Internet 122. All data transferred in a session are encrypted. The analysis server 106 and the feedback data system 124 have fixed private IP addresses.

Thus, the analysis server 106 comprises the AriesteA application 108, the database 112, and the server application 110, which can be used by the processor of the feedback to view and process the data collected by the system. The server application 110 can be implemented for instance as a WWW site to which a connection can be established with the WWW browser 116 over the hospital's Intranet 114. Accordingly, the task of the AriesteA application 108 is to receive the filed transmitted via 104 the FTP from the patient management system 102. The AriesteA application 108 stores the transmitted data in the database 112. The AriesteA application 108 also stores, in the database 112, the enquiry created by the administrator. Thus, the AriesteA application 108 receives patient data from the patient management system 102, transmits the necessary questionnaires and the patient's telecommunication connection identifier to the feedback data system 124 to the AriesteB application 127, and maintains the database 112. The AriesteA application 108 also receives and stores, in the database 112, the replies given by the patient to the reply. The AriesteA application 108 allows the stored data to be browsed and analyzed. The AriesteA application 108 searches for the database 112 for the telecommunication connection identifier of the participant in the enquiry, and possible the web page address (e.g. URL) comprising the enquiry and/or the reply instructions, and transfers it to AriesteB 127 via the RMI 120 once daily. At the same time, it requests, from AriesteB 127, the replies arrived from the patients. The traffic 120 occurs on a daily basis even though no new telecommunication connection identifiers are available, in which case an empty file is transmitted. This way the feedback data system 124 can be sure that the analysis server 106 is operational.

Consequently, the feedback data system 124 comprises the AriesteB application 127, which serves to deliver an enquiry reminder to a patient using the data transmission manner specified by his telecommunication connection identifier, and to receive the replies transmitted by the patients. The replies to the enquiry are transmitted back to the AriesteA application 108 via the AriesteB application 127.

When email is used, the AriesteB application 127 transmits a message via an email server 130 to the patient's email address and possibly displays, via the WWW server 132, the enquiry as an HTML page, to which the patient is able to reply. The AriesteB application 127 receives, via the WWW server 132, the enquiry filled in by the patient. In this case, the patient has access to a computer 152 enabling the set-up of telecommunication links 136, 138 over the Internet 122 to the email server 130 and to the WWW server 132. The AriesteB application 127 is also able to receive a reply to the enquiry via the email server 130 in an email message transmitted by the patient.

The system also enables the use of services offered by the post office that combine a usual letter with an electronic letter. For example, the Finnish Post has two services, eLetter and eCard, which could be used. In this case, the AriesteB application 127 transmits a message via the email server 130 to the patient's mailing address in such a manner that a telecommunication link 154 exists from the email server 130 over the Internet 122 to the post office's server 156. The post office outputs the email arrived at the server 156 and sends it as a usual letter 158 to the patient. The patient replies to the enquiry received using the letter 158. The post office inputs the contents of the letter in its server 156, and transmits the patient's reply, for instance by using FTP, over the Internet 122 to the email server 130, which delivers the reply to the AriesteB application 127. In this case, the patient's telecommunication connection identifier is thus his mailing address, but it is to be noted that the mailing address is not used in the usual manner but in the described manner by combining a usual letter with an electronic letter.

When a short message is employed, the AriesteB application 127 transmits the patient's mobile telephone number to a message server 128. The message server 128 receives the transmitted mobile telephone number of the patient and delivers it over a connection 134 set up over the Internet 122 to a short message centre 142, via which a short message is transmitted to the patient's mobile telephone 150. The enquiry is transmitted for instance in the form: 'Are you going to answer the patient feedback enquiry in accordance with form abc'. The patient writes his reply for instance in the form 1A2C3D4A5A6B7B8C9A or in the form ACDAABBCA and transmits the message back to the short message centre 142, which, correspondingly, delivers the message back via the message server 128 to the AriesteB application 127, which checks the message. Accordingly, the system is able to use the services of a radio system, such as a mobile telephone system 140. The structure of the mobile telephone system 140 is not described in detail herein, but it comprises for instance a base station 144 for establishing a radio connection 146 to a mobile telephone 150.

The system utilizes electronic media because of their real-time and cost effective properties. Having had an appointment at special health care, the patient is either transmitted an electronic enquiry form after a given time to a mobile telephone or, else, the patient receives a link via email to a personally identified reply form. After the form is returned, the results are automatically summed up in a database according to the procedure, its performer, the clinic and the hospital, for example. When a physician wants to browse patient replies, he is to open, vie the Intranet 114, a network site 110 created for this purpose. To gain access to the network site 110, he has to log on to the service using a user id and a password given by the system administrator. The data received as feedback is quantitative, i.e. the reply results therein, regarding properties, can be expressed as figures. The material includes data regarding the same subject about all individuals in the group. As the size of the group under study grows, a large numerical material is easily generated from the data. This is why it may be difficult to distinguish essential data if shown only with figures. Often large statistical material is also shown graphically, by illustrating the properties of the numerical material in the form of suitable statistical graphs. In the study of variables and their interdependencies, graphical presentations are often only indicative. Statistical parameters illustrating the material can be used to clarify them. They sum up, in one figure, the data descriptive of a property and included in the material. The most usual parameters include means and deviations. They serve to measure the average level and variation of the observed values. The replies returned by a patient are automatically summed up according to procedure, its performer or clinic, for example.

After the data are stored, the desired replies may be studied and analyzed in the database 112 via the WWW server 110. The AriesteA application 108 is configured to generate various statistical and graphic presentations descriptive of the feedback collected, which can be studied via the WWW server of the network site/the WWW site 110. An Internet browser (e.g. Microsoft@ Internet Explorer™) may be used to study the feedback.

The information security of the system is guaranteed by placing the analysis server 106 in the hospital premises and inside its information security system. There is no access to patient data from outside. The patient contact data transmitted by the AriesteA application 108 do not contain patient-related identifiable data, and secure traffic exists only from the hospital data system 100 outwards. The hospital data system 102 and the analysis server 108 are protected by the hospital firewall 118. The feedback data system 124 is also protected by a special firewall 126.

The analysis server 106 and the feedback data system 124 can be implemented for instance with computers provided with a Pentium® processor and operating at the Intel@ 1-GHz clock frequency. Both computers contain two 20-gigabyte hard disks and a 265-megabyte main memory. There are two hard disks, since one is reserved for data backup, for instance by means of a RAID driver. The operating system of both computers is Linux Redhat 7.2 and system configuration, i.e. programming is implemented with Java™. Programming is implemented in such a manner that Microsoft@ Windows@ can be used as the operating system in the computers. The computer has to support Java™ and the interfaces used by it, and Web languages, such as HTML, XML and JavaScript.

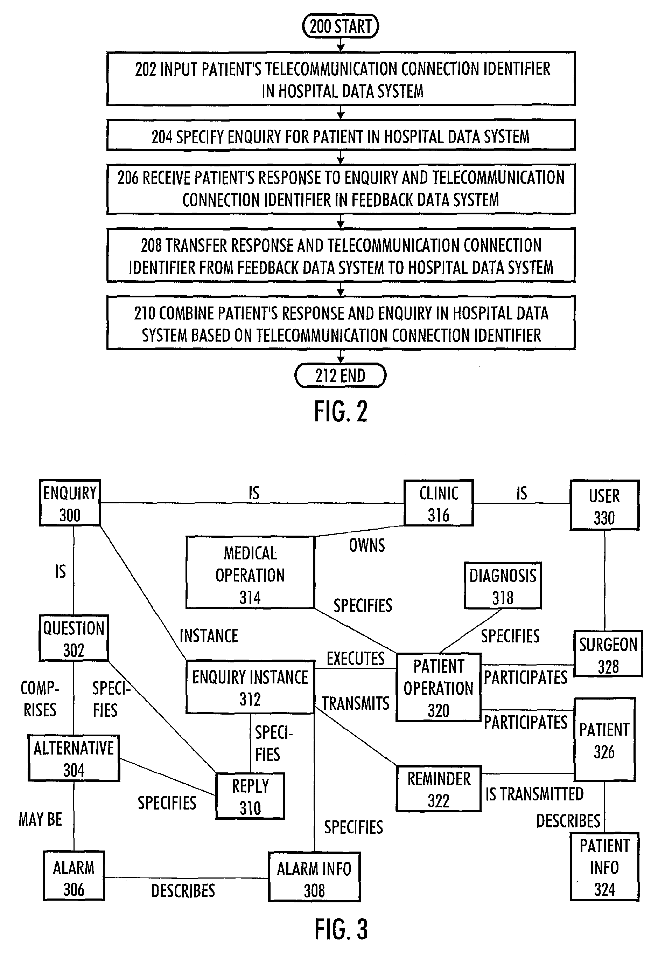

The database 112 of the analysis server 106 can be implemented as a Postgresql database. Data about the hospitals and clinics using the system are stored in the database 112. A special partition is created for the different procedures and related enquiries. Naturally, patient data and replies constitute a group of their own. User (physicians, researchers) data, including user id, password, first name, last name, clinic and health insurance number, are also stored in the database. The structure of the database is shown in Figure 3. It shows array compositions and data storage forms. The lines define the relations between the arrays. The database 129 of the feedback data system 124 can be implemented as a Mysql database. Patient replies to an enquiry are stored in the database 129. Mysql and Postgresql databases are both relational databases, relations based on common fields being defined between the arrays included therein. Dividing the data into different arrays and combining them with each other as relations yields efficient use of space, as common fields do not have to be stored many times. Database integrity is guaranteed by writing difficult street address and code data always in the same manner, for example.

An enquiry 300 includes a varying number of questions 302 and is linked to both a clinic 316 and an enquiry instance 312. The enquiry 300 is always clinic-specific, and the enquiry instance 312 attends to the further processing of the enquiry 300 after the necessary data have arrived.

A given enquiry 300 is linked to a clinic 316. In addition to this, given users 330 belong to the clinic 316, and the clinic 316 is linked to medical procedures 314 according to the clinic. Given users 330 having different access rights belong to the clinic 316 and are able to use their user ids to study the replies 310. Clinic 316 data also include clinic-specific operations. If the system does not have existing clinic-specific operations, the system creates an operation according to the code.

A user 330 is linked to a clinic 316. Depending on his rights, the user 330 is allowed to study the procedures of given surgeons.

A question 302 is dependent on an enquiry 300. The question 302 also includes reply alternatives 302 and the actual reply 310. The questions 302 are included in a given enquiry 300, and special reply models are specified for each enquiry 300.

Alternative 304 is in relation to reply 310 and, when necessary, to an alarm 306. A separate alarm 306 can be connected to an alternative, the alarm serving to transmit a notification about the reservation of a new appointment to the unit that treated the patient or, alternatively, treatment instructions to the patient. The alarm 306 is in relation to alarm information 308 indicative of the type of alarm 306 involved.

A reply 310 is in relation to an enquiry instance 312 and to a question 302, and to an alternative 304. The reply 310 given by a patient 326 is processed before it is input in the database. The reply 310 is also linked to a given question 302.

An enquiry instance 312 is in relation to a reminder 322 and a patient operation 320. In addition, it is connected to a reminder 322 to be transmitted, to alarm information 308 and to a reply 310. The enquiry instance 312 processes the enquiry 300 and the replies 310 that are in relation to a given patient operation 320. The enquiry instance 312 also attends to the transmission of reminders 322 and to the reception of replies 310 and is thus in relation to all message traffic in the system.

A medical operation 314 is connected to a clinic 316 and a patient operation 320.

A patient operation 320 is in relation to an enquiry instance 312, a medical operation 314, a diagnosis 318, a surgeon 328, and a patient 326. A patient operation 320 is a procedure carried out on a patient 326 by a given physician. Hereto is also related a diagnosis 318 preceding the patient operation 320 and constituting the reason why the patient operation 320 is performed.

A reminder 322 is in relation to an enquiry instance 312. A reminder 322 is a request to a patient 326 to participate in an enquiry 300.

A surgeon 328 is in relation to a user 330 and a patient operation 320. The surgeon 328 performs the patient operation 320 and studies the replies 310 returned to the base.

A patient 326 is in relation to patient information 324 and a patient operation 320, and to a reminder 322. A given patient operation 320 is performed on the patient 326, and thereafter a reminder 322 is transmitted at given intervals to participate in an enquiry 300. Patient 326 data are collected as patient information 324, which can be studied later.

Next, a method of collecting patient feedback will be described with reference to Figure 2.

The execution of the method starts in block 200. The patient visits for instance a health centre, where a referral is written for him. The referral arrives at Oys surgical outpatient department, where a physician reads it and evaluates the urgency class of the appointment or operation. Patient data are input in the hospital data system 100 appointment system that gives a suitable appointment time. If a decision to operate is made based on the referral, the appointment is notified to the patient by letter and he receives written instructions about the arrival at the operation. Patient data are transferred to a check-in list and an initial operation program.

In the morning of the day of the operation, the patient arrives at the reception, where he receives a personal data form that he needs to fill in. Based on this information, the patient's data are updated in the hospital data system 100. The social security number serves as the data search key in systems and databases. Patient data are transferred from the initial operation program to a final operation program.

The patient meets a nurse, an anaesthesiologist and a surgeon, and the final decision to operation is made. The nurse presents the system and asks the patient's consent to participating in the system. The consent is recorded in the hospital data system 100. The patient in enquired the telecommunication connection identifier of the data transmission device 148 he uses. In block 202, the patient's telecommunication connection identifier is input 202 in the hospital data system 100. Then, in block 204, an enquiry for the patient is defined in the hospital data system 100.

Patient data are transferred to the final operation program showing e.g. who are to be operated on, the procedure, the operator, the anaesthesiologist, the SSS/DCS patient (short stay surgery/day case surgery) including cancellations. Operation-time data are stored in an operation plan. The patient is transferred to the recovery room. After the operation, the nurse completes a treatment report. In the report, the unit, field of service, surgeon, diagnosis and procedure, for example, are completed using different codes. When leaving, the patient gets along an epicrisis and instructions for further treatment. Together with the outpatient department card, the patient receives an enquiry, reply alternatives and reply instructions. The contents of the reply instructions may vary depending on the telecommunication device 148 used by the patient. A database search is defined in the hospital data system 100 for collecting the necessary patient data such that the social security number, surgeon, diagnosis, procedure and date of procedure are collected from the HILMO, the anaesthesia classification is collected from the LESU, and the patient's telecommunication connection identifier is collected from the address data.

In block 206, the patient's reply to the enquiry and the telecommunication connection identifier are received in the feedback data system 124. Then, in block 208, the reply and the telecommunication connection identifier are transferred from the feedback data system 124 to the hospital data system 100. Lastly, in block 210, the patient's reply is combined in the hospital data system 100 with the enquiry, based on the telecommunication connection identifier. The execution of the method ends in block 212.

The system uses data acquisition resembling the Survey research method. One question does not contain alternative aspects or many items. The wording of the questions is clear and accurate, and the language understandable. The questions of the system are structured, i.e. closed. Therein, the phenomenon being studied and the reply alternatives are known in advance, and the reply alternatives are given to the replier. They facilitate the reply situation and the processing of the replies.

An enquiry may contain for example the following eight multiple-choice questions. The questions were drawn up in cooperation with specialists of the Oys surgical clinic. The contents of the enquiry are modifiable. As the clinics increase in number, the form and focus of the questions change and various enquiries exist. The enquiry to be presented to a patient can be manually defined in the system or then the hospital data system 100 automatically selects the enquiry suitable for a patient based on predetermined criteria, e.g. based on the treating clinic and the operation. An exemplary enquiry:
1. Are you presently experiencing inflammatory syndromes in the area operated (swelling, redness, fever)?
   a) Much
   b) Slightly
   c) Not at all
   d) Do not know
2. Are you presently having a course of antibiotics for an infection caused by the operation (e.g. infection of the operation wound)?
   a) Yes
   b) No
   c) Unable to say
3. Do you presently need a painkiller for the symptom / illness for which you were operated on?
   a) Some times weekly
   b) Daily
   c) More than one painkiller daily
   d) No
   e) Unable to say
4. Are you still on sick leave for the same illness or the operation it caused?
   a) Yes
   b) No
   c) Retired
   d) Not otherwise employed
   e) Unable to say
5. Did the procedure help with your symptom?
   a) Much
   b) A little
   c) Not at all
   d) Made it worse
   e) Do not know
6. Have you had a new doctor's appointment because of the same symptom?
   a) Yes
   b) No
   c) Unable to say
7. Were you satisfied with your treatment as a whole?
   a) Extremely satisfied
   b) Satisfied
   c) Dissatisfied
   d) Do not know
8. If you presently do not have a control appointment agreed with the surgical unit, would you like to be contacted for arranging a control appointment?
   a) Yes
   b) No
   c) Unable to say

In the above, embodiments of the method and system were described wherein a patient's reply is combined in the hospital data system with an enquiry, based on a telecommunication connection identifier. However, this combination can be carried out by the following manners, based on a special enquiry identifier.

In an embodiment of the system, the hospital data system 100 is configured to store the patient's telecommunication connection identifier and the enquiry to the patient, to generate and store an enquiry identifier for the patient's enquiry, and to transfer the patient's telecommunication connection identifier and the enquiry identifier to the feedback data system 124. Such an enquiry-specific enquiry identifier may identify the enquiry. In the generation of the enquiry identifier, the personal identity number and other variables may also be utilized, however, in a manner that makes each enquiry identifier unique to the system. In addition, if the same enquiry is repeatable on a patient several times, an enquiry number identifier may be defined for each enquiry. The enquiry number identifier may be for example an integer whose value is incremented always on transmitting the same enquiry to the same patient. When a specific enquiry is transmitted for the first time to a given patient, the enquiry number identifier may receive the value one, the next time the value two, etc. The enquiry number identifier is stored in the hospital data system 100, and it may also be transmitted to the feedback data system 124.

Furthermore, the feedback data system 124 is configured to transmit, to the patient, via a telecommunication connection according to the telecommunication connection identifier, an enquiry reminder containing at least part of the enquiry identifier, and to receive the patient's reply to the enquiry, and at least part of the enquiry identifier, and to transfer the reply and the enquiry identifier to the hospital data system 100. The hospital data system 100 is also configured to combine the patient's reply with the enquiry, based on the enquiry identifier. Depending on the implementation of the system, the hospital data system 100 may be configured to allow information identifying the enquiry to be decoded from the enquiry identifier, whereby the enquiry is not separately stored for each patient, but only once in the hospital data system 100. Depending on the implementation, during the combination, a check can also be made to see that the enquiry number identifier is valid. Similarly, if the reply contains the patient's telecommunication connection identifier, its validity can be checked during the combination.

In an embodiment, the feedback data system 124 is configured to place at least part of the enquiry identifier in an enquiry reminder in a web page address comprising the enquiry. In this case, the enquiry reminder can be delivered for instance by email to the place defined by the patient's telecommunication identifier. The enquiry reminder is then able to identify the address for instance as an URL, e.g. https://gw.coronaria.fi/spop/main?type=-guest&idcode=pz.9999. In this example, pz.9999 is the enquiry identifier. Thus, a unique address is generated for each enquiry. One way to handle the enquiry number identifier is for the feedback data system 124 to automatically assume that the reply received from a given patient to an enquiry relates to an enquiry that is open in the feedback data system, the enquiry having a given enquiry number identifier that is then added to the reply and transmitted to the hospital data system 100.

In an embodiment, the feedback data system 124 is configured to receive, in connection with a patient's reply, at least part of the enquiry identifier in such a manner that the patient's reply is obtained from the web page whose address includes the enquiry identifier. Consequently, in the above-described manner, at least part of the enquiry identifier may be included in the URL of the web page comprising the enquiry.

In an embodiment, the feedback data system 124 is configured to transmit the enquiry reminder as a text message and place at least part of the enquiry identifier in said text message. In the above example, the URL contained the entire enquiry identifier - pz.9999 - but only part of the enquiry identifier may be transmitted also to a patient. In our example, the enquiry identifier part pz identifies the enquiry, wherefore the text message can include it only.

In an embodiment, the feedback data system 124 is configured to receive, in connection with a patient's reply, at least part of the enquiry identifier in such a manner that the patient's reply is a text message including at least part of the enquiry identifier. In accordance with our example, the text message can thus contain the reply, e.g. in the previously described manner, reply alternatives ACDAABBCA of multiple-choice questions, and enquiry identifier part pz for identifying the enquiry. In this case, the feedback data system 124 may be configured to connect the reply with the enquiry by comparing both the enquiry identifier and the telecommunication connection identifier received from the hospital data system 100 with the at least part of the enquiry identifier and telecommunication connection identifier included in the text message. The comparison of the enquiry identifier or a part thereof is required in cases when the same patient has several different open enquiries, since in that case a comparison of only the telecommunication connection identifiers is insufficient. In addition, the enquiry number identifier can be compared in the feedback data system 124. The final connecting of the reply with the enquiry is then carried out in the hospital data system 100 based on at least the enquiry identifier, but possibly also the telecommunication connection identifier and/or enquiry number identifier.

In an embodiment of the method, the method comprises, not only the previously described blocks 202, wherein the patient's telecommunication connection identifier is input in the hospital data system, and 204, wherein an enquiry for the patient is specified in the hospital data system, but also new blocks and blocks modified from the previously described blocks. Thus, in the method, an enquiry identifier for the patient's enquiry is also generated in the hospital data system, and the patient's telecommunication connection identifier and the enquiry identifier are transferred from the hospital data system to the feedback data system. An enquiry reminder including at least part of the enquiry identifier is then transmitted from the feedback data system to the patient using a telecommunication connection according to the telecommunication connection identifier. The patient's reply to the enquiry and at least part of the enquiry identifier are then received in the feedback data system. The reply and the enquiry identifier are then transferred from the feedback data system to the hospital data system. Lastly, the patient's reply is combined with the enquiry in the hospital data system, based on the enquiry identifier. In an embodiment, in the enquiry reminder, at least part of the enquiry identifier is contained in the address of the web page comprising the enquiry. In an embodiment, at least part of the enquiry identifier is received in connection with the patient's reply in such a manner that the patient's reply is obtained from the web page whose address includes at least part of the enquiry identifier. In an embodiment, the enquiry reminder includes a text message including at least part of the enquiry identifier. In an embodiment, the reply includes a text message including at least part of the enquiry identifier. In addition, the method can be modified by the above-described system-related specifications.

Although the invention is described above with reference to the examples according to the attached drawings, it is apparent that the invention is not limited thereto, but can be modified in a plurality of ways within the inventive idea disclosed in the appended claims.

## Claims

1. A method of collecting patient feedback, **characterized by** comprising:
inputting (202) a patient's telecommunication connection identifier into a hospital data system;
specifying (204) an enquiry for the patient in the hospital data system;
receiving (206) the patient's reply to the enquiry and the telecommunication connection identifier in a feedback data system;
transferring (208) the reply and the telecommunication connection identifier from the feedback data system to the hospital data system; and
combining (210) the patient's reply with the enquiry in the hospital data system, based on the telecommunication connection identifier.

2. A method as claimed in claim 1, **characterized by** the method further comprising: transferring the patient's telecommunication connection identifier from the hospital data system to the feedback data system.

3. A method as claimed in claim 1, **characterized by** the method further comprising: transmitting an enquiry reminder from the feedback data system to the patient using a telecommunication connection according to the telecommunication connection identifier.

4. A method as claimed in claim 3, **characterized by** the enquiry reminder including a link to a web page comprising the enquiry.

5. A method as claimed in claim 3, **characterized by** the method further comprising: transferring the contents of the enquiry reminder from the hospital data system to the feedback data system.

6. A method as claimed in claim 1, **characterized by** the method further comprising: receiving, in the feedback data system, the reply given by the patient on a web page.

7. A method as claimed in claim 1, **characterized by** the telecommunication connection identifier being an email address and the method further comprising: the patient transmitting the reply in an email message to the feedback data system.

8. A method as claimed in claim 1, **characterized by** the telecommunication connection identifier being a radio system subscriber number and the method further comprising: the patient transmitting the reply in a short message to the feedback data system.

9. A method as claimed in claim 1, **characterized by** the method further comprising: giving the enquiry, reply alternatives and reply instructions to the patient during a hospital visit.

10. A method as claimed in claim 1, **characterized by** the method further comprising: delivering, to the patient, an address to a web page where the enquiry, reply alternatives and reply instructions are found.

11. A method as claimed in claim 1, **characterized by** the reply being composed of symbols identifying the selected reply alternatives.

12. A method as claimed in claim 1, **characterized by** the data transmission between the hospital data system and the feedback data system being initialized by the hospital data system along a secure and/or encrypted telecommunication connection.

13. A system for collecting patient feedback, **characterized in that** the system comprises:
a hospital data system (100) configured to store a patient's telecommunication connection identifier and an enquiry to the patient; and
a feedback data system (124) configured to receive the patient's reply to the enquiry and the telecommunication connection identifier, and to transfer the reply and the telecommunication connection identifier to the hospital data system (100); and
the hospital data system (100) is further configured to combine the patient's reply with the enquiry, based on the telecommunication connection identifier.

14. A system as claimed in claim 13, **characterized in that** the hospital data system (100) is further configured to transfer the patient's telecommunication connection identifier to the feedback data system (124).

15. A system as claimed in claim 13, **characterized in that** the feedback data system (124) is further configured to transfer an enquiry reminder to the patient by using a telecommunication connection according to the telecommunication connection identifier.

16. A system as claimed in claim 14, **characterized in that** the feedback data system (124) is further configured to add, in the enquiry reminder, a link to a web page comprising the enquiry.

17. A system as claimed in claim 14, **characterized in that** the hospital data system (100) is further configured to transfer the contents of the enquiry reminder to the feedback data system (124), and the feedback data system (124) is further configured to receive the contents of the enquiry reminder from the hospital data system (100).

18. A system as claimed in claim 13, **characterized in that** the feedback data system (124) is further configured to receive the patient's reply from a web page.

19. A system as claimed in claim 13, **characterized in that** the telecommunication connection identifier is an email address, and the feedback data system (124) is further configured to receive the patient's reply in an email message.

20. A system as claimed in claim 13, **characterized in that** the telecommunication connection identifier is radio system subscriber number, and the feedback data system (124) is further configured to receive the patient's reply in a short message.

21. A system as claimed in claim 13, **characterized in that** the hospital data system (100) is further configured to output the enquiry, reply alternatives and reply instructions.

22. A system as claimed in claim 13, **characterized in that** the feedback data system (124) is further configured to deliver, to the patient, an address to a web page where the enquiry, reply alternatives and reply instructions are found.

23. A system as claimed in claim 13, **characterized in that** the hospital data system (100) is further configured to generate the enquiry such that the enquiry is composed of multiple choices, and the reply is composed of symbols identifying the selected reply alternatives.

24. A system as claimed in claim 13, **characterized in that** the hospital data system (100) is further configured to initiate data transfer with the feedback data system (124) along a secure and/or encrypted telecommunication connection.

25. A method of collecting patient feedback, **characterized by** the method comprising:
inputting a patient's telecommunication connection identifier in a hospital data system;
specifying an enquiry for the patient in the hospital data system;
generating an enquiry identifier for the patient's enquiry in the hospital data system;
transferring the patient's telecommunication connection identifier and the enquiry identifier from the hospital data system to a feedback data system;
transmitting an enquiry reminder including at least part of the enquiry identifier from the feedback data system to the patient by using a telecommunication connection corresponding to the telecommunication connection identifier;
receiving the patient's reply and at least part of the enquiry identifier in the feedback data system;
transferring the reply and the enquiry identifier from the feedback data system to the hospital data system; and
combining the patient's reply with the enquiry in the hospital data system, based on the enquiry identifier.

26. A method as claimed in claim 25, **characterized by** at least part of the enquiry identifier in the enquiry reminder being included in an address of a web page comprising the enquiry.

27. A method as claimed in claim 25, **characterized by** receiving, in connection with the patient's reply, at least part of the enquiry identifier in such a manner that the patient's reply is obtained from the web page whose address includes at least part of the enquiry identifier.

28. A method as claimed in claim 25, **characterized by** the enquiry reminder comprising a text message including at least part of the enquiry identifier.

29. A method as claimed in claim 25, **characterized by** the reply comprising a text message including at least part of the enquiry identifier.

30. A system for collecting patient feedback, **characterized in that** the system comprises:
a hospital data system (100) configured to store a patient's telecommunication connection identifier and an enquiry to the patient; to generate and store an enquiry identifier for the patient's enquiry, and to transfer the patient's telecommunication connection identifier and the enquiry identifier to a feedback data system (124);
a feedback data system (124) configured to transmit an enquiry reminder including at least part of the enquiry identifier to the patient by using a telecommunication connection corresponding to the telecommunication connection identifier, and to receive the patient's reply to the enquiry and at least part of the enquiry identifier, and to transfer the reply and the enquiry identifier to the hospital data system (100); and
the hospital data system (100) is further configured to combine the patient's reply with the enquiry, based on the enquiry identifier.

31. A system as claimed in claim 30, **characterized in that** the feedback data system (124) is configured to place, in the enquiry reminder, at least part of the enquiry identifier in the address of a web page comprising the enquiry.

32. A system as claimed in claim 30, **characterized in that** the feedback data system (124) is configured to receive, in connection with the patient's reply, at least part of the enquiry identifier in such a manner that the patient's reply is obtained from the web page whose address includes at least part of the enquiry identifier.

33. A system as claimed in claim 30, **characterized in that** the feedback data system (124) is configured to transmit the enquiry reminder as a text message and place at least part of the enquiry identifier in said text message.

34. A system as claimed in claim 30, **characterized in that** the feedback data system (124) is configured to receive, in connection with the patient's reply, at least part of the enquiry identifier in such a manner that the patient's reply is a text message including at least part of the enquiry identifier.
